# EUROPEAN PATENT APPLICATION

(11) **EP 2 816 495 A1**
(43) Date of publication of application: **24.12.2014**
(21) Application number: 14173256.0
(22) Date of filing: 20.06.2014
(51) Int. Cl.: G06F 19/00

(54) **Apparatus for managing an operating room**

(30) Priority: 20.06.2013 RU 2013128248
(71) Applicant: Kazinov, Vladimir Aleksandrovich, 107140 Moscow (RU)
(72) Inventor: KAZINOV, Vladimir Aleksandrovich, 107140 MOSCOW (RU); DANILOV, Andrey Borisovich, 105122 MOSCOW (RU); POKROVSKIY, Konstantin Pavlovich, 115184 MOSCOW (RU); FEDOROV, Vladimir Valentinovich, 142407 MOSCOW (RU)
(74) Representative: Girlando, Mario

(57) **Abstract**

An apparatus for managing an operating room, comprises a core unit connectable to at least a medical unit and/or at least an engineering unit in the integrated operating or hybrid room of the healthcare institution, the core unit is configured to output at least a video signal and at least a data signal; synchronous multi-track registration means connected to the core unit and configured to record and transmit the data/and or the video signal; a plurality of display units, each connected to the core unit and configured to display the video signal and/or a visual representation of the data signal in one or more display windows.

## Description

The present invention relates to an apparatus for managing an operating room. The apparatus is particularly useful in the field of medicine and can be used in operating or hybrid room.

The basic idea of the integrated solutions of medicine is the following.

In any operating or hybrid room there used different medical and engineering equipment and devices, as well as information and engineering systems:
- medical equipment, the output of which is an analogue or digital video. This can be video camera of operating field, endoscope video camera, video camera of operating microscope, operating room general view video camera, the video signals from the ultrasound systems, X-ray or angiography systems, etc.;
- medical devices, the output of which data are available. These are patient monitor, anesthesia machines, the infusion therapy station, heart-lung machines, ventilator, medical analyzers, etc.;
- engineering systems that create a sterile area and provide a sustainable environment in the operating room. The output of the sensors of these systems contains information about power supply, temperature, humidity, air purity, flow and stock of various gases, etc.;
- medical information systems that contain electronic patients records with a history and clinical data, the results of various medical procedures performed on the patient, such as radiology images in the PACS (Picture Archiving and Communication System - information system based on DICOM standard for medical images), or the results of previous tests, etc.

In the Integrated Operating Room (the IOR) all kinds of information from the above medical and engineering appliances and equipment, and clinical information systems are quickly available, to physicians and nurses of the surgical team, as well as to physicians or health care managers, who are outside the IOR, with against their right of access to some information.

The analogs of the hardware and software for the integrated operating or hybrid room of the healthcare institution:
1. Karl Storz OR1, Germany (https://www.karlstorz.com/cps/rde/xchg/SID-F4AF505B-06347A2E/karlstorz-ru/hs.xsl/522.htm). However, this system though is called integrated, but made only for endoscopic operating rooms and endoscopy only for their own production and at its core is a centralized management system for some equipment of the operating room. No data exchange capabilities with medical appliances, no possibility of a multi-track recording, no possibility of integration with the Hospital Information System (HIS), Radiological Information System (RIS), and Laboratory Information System (LIS), making it impossible to create a full integration of all types of information that are used during operation.
2. Maquet TEGRIS Co., Germany (http://www.maquet.com/productPage.aspx7m1=112599774495 &languageID=1&title CountryID=176&productConfigID=133285429284&productGroupID=117128272834 ). The system is designed for transmission and recording of audio and video information. There is no possibility of data exchange with medical appliances, there is any possibility of a multi-track recording, there is no possibility of integration with the HIS, RIS, and LIS, that does not provide physicians with relevant information for the local and remote decisions.
3. Richard Wolf Core Co., Germany (http://www.richard-wolf.com/en/core.html). However, this system though is called integrated, but made only for the endoscopic operating rooms and endoscopy only for their own production and at its core is a centralized management system for some equipment of the operating room. No data exchange capabilities with the medical appliances, no possibility of a multi-track recording, no integration with the HIS, RIS, and LIS, making it impossible to create a full integration of all types of information that are used during the operation.
4. Olympus EndoAlpha, Japan (http://www.olympus.com.ru/medical/en/medical_systems/products_services/systems integration/productselector_service_solutions_8.jsp). However, this system though is called integrated, but made only for the endoscopic operating rooms and only for endoscopes of their own production, and at its core is a centralized management system for some equipment of the operating room. No data exchange capabilities with medical devices, no possibility of a multi-track recording, no integration with the HIS, RIS, and LIS, making it impossible to create a full integration of all types of information that are used during the operation.

Reached at realization of offered system the medico-technical result is in fully integration of medical and technical means of an operational rooms that provides obtaining all information on the actions made during operation, at the expense of collecting, processing, the analysis, recording, storage, transfer and reception of data, and also remote consultation and medical training.

### SUMMARY

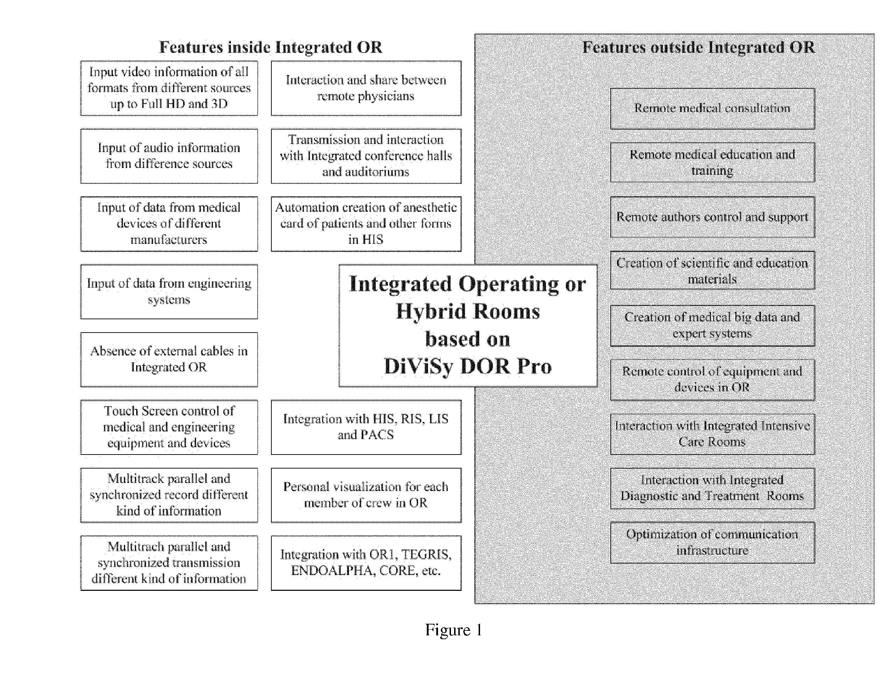

An integrated operating room (IOR) has advantages over conventional or hybrid operating rooms due to the presence of hardware and software system. Further the main opportunities and the IOR functions, both in a rooms, and out of its, in comparison with usual operational or hybrid rooms are given. Due to a hardware and software system, the integrated operation room system has advantages compared to conventional operational or hybrid rooms (see Figure 1). 1. Video information from various medical equipment and devices.

The hardware and software system of IOR can receive, process, record and transmit all analog or digital video information from the various video cameras (video camera of operating field, forehead camera, endoscope video camera, video camera of operating microscope, operating room general view video camera, etc.) and medical equipment (angiographic and X-ray systems, ultrasound systems, electrophysiological devices). As an interface may be used, for example, the analog interfaces of Composite, S-Video, RGB, VGA/DVI, etc., or digital interfaces DVI, SDI, HDMI, etc., or non-standard interfaces, for example in the angiographic and X-ray systems, included FullHD 1080P/50/60.

### 2. Audio information

The hardware and software system of IOR, in addition to the video information, can receive, process, record and transmit the audio signals from different microphones (wireless microphones, wired microphones, built-in microphones), as well as play audio information to various audio systems of operating rooms, conference rooms, training rooms, and personal working places of physicians.

### 3. Data from various medical devices.

The hardware and software system of IOR can receive, process, register and transmit data from various medical devices which are used in the course of carrying out operations: patient monitors, anesthesia machines, ventilators, heart-lung machines, various analyzers, infusion therapy stations, and other medical devices, regardless of the types and manufacturers are recorded and translated using the hardware and software system, which can also obtain information from various sensors of engineering systems, ensuring the functioning of operating rooms: the temperature, humidity, gas flow, voltage, and others. 4. Data from sensors of engineering systems.

The hardware and software system of IOR can receive, process, register and transmit data from various sensors of the engineering systems providing functioning of operational rooms: temperatures, humidity, consumption of gases, power supply, and others. All these data are transferred, displayed and registered synchronously and in parallel with other types of information. 5. Personal visualization.

Individual display can be set for each member of the surgical team in the IOR. Individual displays are located on the special sets and mounts of pendants and lamps and can change position depending on the medical operations technology. Each member of the surgical team can receive information that he needs just in time, to the individual display. For example, the main operating surgeon can see information from a video camera of operation field or endoscopy camera, the data from the patient monitor, X-ray images, etc., the anesthetist can see data from the patient monitor and from the anesthesia machines, the clinical information from the HIS, RIS and PACS, etc., the perfusionists can see data from patient monitor, the blood test results on gases and electrolytes, and data from the infusion therapy station, etc., video information about surgeon activities can display for the nurses in real time. Personal visualization allows each member of the surgical team to place the types of information that he needs at any given point in time, on the screen. All display control information is carried out with a Touch Screen display (TSs), and one or other information can quickly navigate between different windows of a display and from one display to another. This feature is extremely important in the case of operations of visiting surgeon, who can show the unusual for the healthcare institution requirements on access to the information.

### 6. Fast access to different types of information

The IOR can quickly display on different displays the information about the current events in the operating room, as well as the information from test results, diagnostic and therapeutic procedures performed earlier for the patient from HIS, RIS, and PACS. Any member of the surgical team can receive the types of information that he needs for the quality work, in any window on its personal display. Any medical information can quickly navigate from one screen to another and from one display window to another. Information obtained in the course of the operation, can also be transferred to the HIS, RIS, and PACS.

With more detail, video information can be received from different medical equipment with video outputs (video cameras in the surgery lamps, endoscopic video cameras, observing video cameras, surgery microscopes video cameras, video from ultrasound equipments, video from C-Arms, X-Ray equipment, etc.). Additionally, the system can manage data from medical devices (monitors of patients, narcosis machines, ventilators, stations of therapy, etc.), data from engineering systems (power supply, temperature, humidity, medical gases capacity and fuel, etc.). More generally, all of these information can be synchronized, transmitted and recorded by the DiViSy DOR unit receiving in real time.

All the processes for requesting and obtaining information are recorded and stored. For example, the following scheme may be implemented: the blood test to gases and electrolytes is done periodically during time of cardiac surgery with use of heart-lung machines. The tube with blood shall be sent to the laboratory and analyzed there. The result of the analysis is delivered promptly to the perfusionist's personal display in the IOR, and he does the correction of the therapy and cardiopulmonary bypass modes. All stages of this process, with the results of the analysis and modes correction, are registered and can be completely remodeled after the operation.

### 7. Record of all types of information in the course of carrying out surgery operation.

In IOR, multi-track, synchronous record of different types of the above medical information is made: video of operational field, video from the endoscope, video of a general view of an operational room, image from ultrasound systems, from the angiograph or other medical equipment having analog or digital videos interfaces, audio information, data from various medical devices (monitor of the patient, the narcosis machine, therapy station, etc.), and information from sensors of engineering systems (temperature, humidity, expenses of gases, etc.).

The situation that was in the IOR at any time can be completely reconstructed based on the multi-track record. On the basis of multi-track records performed, both full illustrated and dynamic standards, flow diagrams of carrying out various operations and their key moments, manuals and scientific materials, as well as databanks for the subsequent creation of expert systems can be created.

### 8. Independence from types of medical and engineering equipment and devices.

The medical equipment and appliances of virtually all manufacturers can be connected to the hardware and software system of IOR. Medical and engineering equipment and devices must have physical interfaces and descriptions of the data communications protocols. 9. Integration with various control systems of operational rooms.

The hardware and software system of IOR can be integrated with the centralized control system of operating rooms, such as Karl Storz OR1, Olympus ENDOALFA, Richard Wolf CORE, Maquet TEGRIS, and others. Despite the fact that the manufacturing companies of the centralized control systems call them integrated operating rooms, as such they are not, because they cannot fully provide integration between medical devices and equipment, engineering systems, and various information systems.

Further details and specific embodiments will refer to the attached drawings, in which:
- Figure 1 is a diagram of the functions performed by the apparatus according to the invention.

### DETAILED DESCRIPTION

The following description of exemplary embodiments refer to the accompanying drawings. The same reference numbers in different drawings identify the same or similar elements. The following detailed description does not limit the invention. Instead, the scope of the invention is defined by the appended claims.

Reference throughout the specification to "one embodiment" or "an embodiment" means that a particular feature, structure, or characteristic described in connection with an embodiment is included in at least one embodiment of the subject matter disclosed. Thus, the appearance of the phrases "in one embodiment" or "in an embodiment" in various places throughout the specification is not necessarily referring to the same embodiment. Further, the particular features, structures or characteristics may be combined in any suitable manner in one or more embodiments.

The apparatus comprises at least a core unit, which is connectable to at least a medical unit and/or at least an engineering unit in the integrated operating room of the healthcare institution. Indeed, the core unit is configured to output at least a video signal and at least a data signal.

All the medical equipment and devices in the operating room are connected to the core unit through a cable system, which is conducted inside the pendants behind suspended ceilings and walls of the IOR, with no external cables inside IOS. The core unit is located outside the IOR clean zone. In some cases, the wireless transmission of the information from the medical devices and equipment to core unit, including wireless transfer of the FullHD quality video information, can be used. The free movement of the members of the surgical team in the IOR, without risk to catch some of the cables, optimizes the operation time.

With additional detail, the apparatus comprises synchronous multi-track registration means connected to the core unit and configured to record and transmit the data/and or the video signal. The core unit can also be adapted to perform the synchronous functions of the multi-track synchronous recorder which simultaneously records the video information in parallel from the medical device, the data from the medical appliances, engineering systems, types, and time of arrival of information from the HIS, LIS, RIS systems, and the PACS, wherein the recorded information is then placed in the PACS, or in the central repository of the healthcare institution, and the reference to it is placed in the electronic patient record.

The apparatus also comprises a plurality of display units, each connected to the core unit and configured to display said video signal and/or a visual representation of the data signal in one or more display windows.

Preferably, the core unit is configured to acquire at least an input signal from the medical unit and/or the engineering unit. The core unit also comprises a data elaboration module configured to acquire the input signals, extract at least an input value from each of said data signals and to compute at least an output value. The data signal represents the output value.

With additional detail, the core unit also comprises a control module configured to output a control signal for at least one of either medical unit and/or display unit and/or engineering unit.

The core unit can connect to any of the healthcare information system (HIS), radiology information system (RIS), laboratory information system (LIS), and the Picture Archiving and Communication System (PACS) by the HL7 and Dicom 3.X protocols. This integration makes it possible, for example, to add an electronic patient record with the diagnostically useful information, such as static and dynamic image information, from medical equipment and devices, the results of remote consultation, etc. Even if the HIS and RIS do not support the HL7 protocol, integration can be carried out based on the XML protocol or a specially developed software module, converting data formats for the exchange of information. Additionally, the core unit is connectable to hardware and software system of physicians and/or consultants, experts, health care managers, training rooms and conference rooms.

Automation of the individual components of the clinical process, for example, autocomplete of narcosis patient card, is now possible in the IOR. Formerly, it used to be filled in the form of records on the paper, then the information was entered from the keyboard into PC, which increases the complexity of the work of physicians and there is the possibility of errors associated with the human factor. In the IOR, information from the patient monitor (and from other medical devices) is automatically sent to the electronic patient record in the HIS at intervals to be established by physicians before or during surgery operation. Such intervals can promptly change depending on the situation in the operating room. After the surgery, physician views the electronic patient record from the personal workplace. If he it sees, for example, that the condition of the patient has changed for the worse at some point in time, he it presses the "View Operations" button and automatically starts a multi-track player that plays the operation recording since that time. There are "hour", "minute", "second" buttons, located at the interface of the player, using which physician can view the previous information and determine the time and cause of the deterioration of the patient's health. This information is objective, because it is prepared without the influence of the human factor. The automated filling of other reporting forms can be also performed in the same way.

All information is transmitted from the IOR in the meeting rooms and classrooms, including those outside the medical institutions. This is used for the interactive distance education of young physicians and students as well as for carrying out the interactive workshops for unique surgeries. After the sessions of distance learning, and workshops, multi-track recording operation can be transferred to the external media in the form of education or scientific materials.

In the course of carrying out surgery in the IOR, the transmission of all kinds of information on personal medical facilities outside the IOR is provided for distance education and consultation. Because of this, the probability of occurrence of an insured event is reduced, including as by monitoring and assistance from more experienced surgeons, and due to remote consultation of the physicians of various specialties, in the event of the occurrence of the unexpected events that are not directly related to the surgical zones. There is the possibility of intraoperative consultation of histology and cytology during the cancer surgeries, with the participation of pathologists outside the IOR, including the possibilities of the robotic microscopes and scanners remote control. In the process of the remote consultation, colored drawing, marking areas of the medical images, text and sound transmission convenient tools are used. All the results of counseling are placed in the PACS system or in the electronic patient records.

The IOR has a function of the remote medical and engineering equipment control having the functions of this control: video cameras, endoscopes, electrosurgical, microscopes, operating light of lamps and general lighting, patient table, blinds, etc.

All the control in the IOR is performed by using of the interface on the Touch Screen Display (displays). This control allows you to quickly switch the information from one display to another, request additional types of information from the HIS, RIS, and the PACS, manage the process of recording and translation, as well as to change remote control interfaces of the medical equipment. Pressing the different buttons on the Touch Screen display is made with your fingers, including the fingers in the surgical gloves.

The presence in the IOR of communication modules allows you to organize the optimal structure of the flow control of medical information between the IOR and remote complexes that provides reliable and quality performance of the data networks, which comprise the integrated systems for operating and/or hybrid rooms. The medical information, to the IOR during various operations, allows its subsequent detailed study, analysis and creation of the illustrated and dynamic standards, flow diagrams, guidelines and expert systems to support physicians in making the right decisions. The standards created are not just a text document, but reference record of all medical procedures that can be played on one or more IOR displays during similar manipulations in frame of surgeries. Thus, the physicians are able to correct their actions in the real time, focusing on standards that simultaneously show properly conducted similar medical procedures. At the same time, expert systems will help, for example, when transferring a patient from the cardiopulmonary to natural bypass, and a number of other cases that require an on-line analysis of large amount of various medical information to make good decisions.

It becomes possible to perform the remote authorized supervision over the implementation of new medical technologies, equipment and drugs in the IOR. As a rule, introduction of the new technologies and products is accompanied by the preliminary training and conduct of master classes. These processes are an integral part of the IOR, and described above. Further, there is a good reason for the creators of these technologies, equipment, prostheses and drugs to periodically remotely participate in the procedures for their use, to provide advices on possible conflicts and improve the skills of practicing physicians.

In the IOR, every physician of the surgical team can get medical consultation, and render it to other physician, just in time when it is needed. It allows to create quality of carrying out operations and to optimize time of their carrying out. Subsequent analysis of the medical information in the study of the recorded surgery operations allows you to find mistakes in the actions of medical or other personnel, and to create the necessary illustrated instructions to avoid similar mistakes in the future. The technical result achieved by the proposed hardware and software system in an integrated or hybrid operating room means optimization of the flow control of medical information, timely delivery of relevant information to each member of the surgical team, enhancing the quality and efficiency of medical operations through the ability to obtain complete information about the actions performed in the operation, through the collection, processing, analysis, recording, storing, transmitting and receiving data, remote consultation of physicians in the real-time operations, medical education based on the analysis of data obtained as a result of operations, optimization of the hardware and software system through the ability to connect it to a different type of equipment.

The technical result of the claimed invention is achieved by creating hardware and software system for receiving, synchronous, multi-track registration, recording and transmission of the various medical video and audio information as well as data from the medical and engineering devices in the healthcare institution's operating room, which is connectable via cables to medical equipment, at the output of which the video data is formed, with the video displays in the operating room for demonstrating in one display window as well as in several display windows, and a through cables or sensors of medical devices, the output of which the individual data are formed, as measured by means of these devices, data and through the cables and sensors with engineering systems, providing and maintaining the operating parameters of the microclimate of the room, forming values of these parameters at the output, while the medical devices, equipment, displays, and engineering systems have the ability of remote control using the remote or other local control devices, via the healthcare institution data network with the Healthcare Information System (HIS), containing electronic patient records, with the Laboratory Information System (LIS), which contains the results of laboratory tests, and the Radiological Information System (RIS), containing the static and dynamic images obtained by the use of the different diagnostic techniques during patients' study with the PACS, with a central information repository of the healthcare institution, with the hardware and software system of the physicians, consultants, experts, health care managers, training rooms and conference rooms. The core unit is capable of simultaneous multi-track recorder function which implements the synchronous and parallel video recording from the medical equipment, the data from medical devices, engineering systems, types and time of information arrival from the HIS, LIS, RIS systems and the PACS, wherein the recorded information is then placed in the PACS or in a central repository of the healthcare institution, and the reference to it is placed in the patient's electronic medical record, it is adapted to perform the functions of synchronous multi-track player, which synchronously and simultaneously reproduces previously recorded operation, containing information from the medical equipment, medical devices, engineering systems, as well as the types and time of information arrival from the HIS, LIS, RIS and the PACS; is capable of automatic and semi-automatic filling of reporting forms required for a particular type of operation; is capable of backup control of medical equipment and devices, displays, engineering systems; is capable of the multi-track nonlinear editing function for creating education and/or scientific materials and/or medical flow diagrams and/or standards for surgical or hybrid operations.

Examples of the use of the integrated system of an operating or hybrid room using the hardware and software system:

In the operating room, there is an operating table, anesthetic equipment, heart-lung machine, and patient monitor, operating light and other necessary equipment. The control units of operating lamps video cameras, headlamp cameras and/or operating microscopes cameras, the endoscopic, or other surgical or anesthetic equipment is placed on pendants. The operating surgeon (or physicians) sees the surgical site, performs the necessary surgical operations on the patient, monitors the position and operation of each member of the surgical team, independently and/or through assistants and anesthesiologists monitors he controls the status of the patient; all by himself or by assistants, he controls the light, the position of patient table, etc. During the operation there is voice communications between members of the surgical team, as well as scrolling the additional information: clinical data and X-ray pictures, etc.

The members of the surgical team, in accordance with their official duties, record certain readings of medical equipment, usually by using the key points of the operation plan. In a normal operating room this registration is filling the log sheet of the patient, the protocol of the anesthetic equipment readings, etc.; in an integrated operating room this goes automatically. On the surgical pendant there is everything you need to connect the various surgical equipment. On the anesthetic pendant, there is all you need to access the anesthesia equipment, monitoring equipment, and the functional diagnostics. On the bracket, a Touch Screen display is installed, with your control and supervision over the work of the integrated system in the operating room. With more detail, the individual displays are located on the outboard brackets and are adapted to change the location, as well as in a wall or the shelves of the health pendants.

Specifically, personal displays for each member of crew in OR are located on the surgery lamp arms, pendant arm or shelves, wall arms or on other places. It depends from surgery management requirements. Displays on the arms may to change of positions like prefer physicians.

With this display, you can manage working with a variety of medical images, control the light fixtures and the total light in the operating room, scroll, and analyze the readings of the anesthetic equipment, infusion therapy station, and other life support equipment, etc., as well as call any other digital operating room or personal complexes of physicians both in the clinic, and beyond. The various medical information, necessary for the assistants, anesthesiologists, perfusionists, and nurses, is shown on the other high-resolution displays.

Interaction with the PACS system can be based on several schemes. First, the principle of viewing the images received from the PACS system at one of the displays of hardware and software, without the use of processing capacity, and reconstruction of images, which are available in the workstations of the PACS. It is most in demand in the operating room, as during surgery operation there is neither need, or time for any manipulation of the radiology images, and, basically, you need only to view them. Second, the startup mode of the remote hardware and software system of the PACS system's workstation interface with subsequent processing of the medical images. This mode is in demand, for example, in consultation with the remote physicians. And, the third mode is the mode of interaction of the remote hardware and software system with the PACS system through the health information system. In this case it is possible to obtain images of the PACS system in conjunction with the clinical information. All these modes are implemented within the scope of the remote hardware and software system. The surgeon can always give advice to a physician who is in a remote integrated operating room, even during surgery. Local or remote medical information may be displayed differently on displays. For example, if physicians (a surgeon and his assistant) stand one against the other during the operation, in the front of each physician there is a display with the same medical information which is an image from the endoscope video camera. In the other case, when a visiting doctor carries out the operation, and the operating team has no experience with it, one of the displays showing the operating field, is positioned so that assistants or surgical nurses can observe the process of the operation, and timely respond to the demands of the surgeon. The ability to navigate between different displays of various processes of medical information meets the requirements of different scenarios of the operation. On any of the displays directly in the operation, the surgeon may call the radiology images of the patient from the PACS system obtained before the operation, as well as the necessary clinical information from the medical information system.

All the medical equipment and displays are placed on pendants and brackets. Inside the pendants and the brackets, power cables, informational and video cables, as well as distortion and crosstalk suppression devices are located. Integrated operating room has another significant advantage, i.e. there are no cables that restrict the movement of the members of the surgical team. There is a controlled (zoom) camera on the main lamp to display the surgical site. The dome camera, located on the ceiling in the operating room, is for surveillance and recording of processes carried out in the operating room. In the operating room near the equipment room, there is a set of the hardware and software system that all the information and video cables can be connected to. Number of displays in the operating room may be decreased or increased. Reducing the number of displays in a certain way limits the functionality of the integrated operating room, namely, instead of a parallel display of the various types of medical information on the various displays, the input mode while sequential browsing the images on one or two monitors, is introduced. This increases the number of manipulations on the control display, which the physician must perform a during surgery, and may slow the response time of the assistants or nurses to the requirements of the surgeon, because of limitations of visual control over what's happening.

Various medical equipment is controlled using a Touch Screen display (displays). This control does not by itself discharge the functions of the local or remote control, which is mounted on some medical equipment, and just backs it up. For example, if the operating table is controlled with an infrared remote control, the control display has an interface with buttons that duplicate all the functions of the IR remote control, and the physicians can operate the surgical table both with the IR remote control, and with the control display. Similarly, on the control display are shown the interfaces to work with the different medical equipment, copying existing interfaces.

The hardware and software system of the integrated operation or hybrid room performs continuous synchronous recording of all streams of the medical information that are available in the digital operating room, namely, multiple video streams of the medical information, multiple streams of the audio information, and multiple streams of the data from the medical devices. All medical equipment control signals are also registered.

It is possible to record information accompanied by markers. These markers serve for, a quick search and scrolling the most important fragments of the record after the operation is carried out. Simultaneous registration of the various types of the information: video depicting the surgical site, the video showing the general view of the operating room, voice of local and remote physicians (staff), data from the anesthesia equipment, patient monitor, infusion therapy stations, heart-lung machine, etc., can fully restore the situation that was in the operating room at any given time. Exchange of information of the hardware and software systems with the medical equipment is carried on a variety of digital interfaces such as RS-232, RS-485, USB, LAN, VGA, DVI, SDI, HDMI, etc. All of these interfaces are included in these hardware and software systems. To make possible the exchange between the hardware and software systems and the medical equipment, the software modules with the corresponding friendly interface are created. For instance, the fluid therapy station registers several parameters characterizing the modes of its work in the course of operations. These parameters are accumulated for a certain time, for example, the day, and can be prepared in the form of a report as per working day or during a particular operation. In addition, the software module gives a possibility to establish the periodicity of registration of the basic parameters: every second, every five seconds, etc., depending on the nature of the operation. Similarly, the basic parameters of the patient monitor and anesthesia equipment are defined, as well as the frequency of their synchronous recording, and some other types of the medical information. If the operation is normal and the condition of the patient during surgery is adequate, there is no need for frequent check, for example, of the pressure or heart rate indications. However, if the patient condition becomes inadequate as for the level of surgical intervention, the frequency of registration can be rapidly increased up to several times per second. All these parameters can be set in code modules, in a planned manner, as well as in an emergency mode. Once the operation is completed, there is a possibility of automatic production of a DVD or another disc (one or several, depending on the duration of the operation). All the recorded multichannel information is copied to the disc. Further, if necessary, the nonlinear editing workstation performs playback, including based on the markers, and prepares samples of the most significant information fragments for the creation of the informational material to be a long-term storage.

## Claims

1. Apparatus for managing an operating room, comprising a core unit connectable to at least a medical unit and/or at least an engineering unit in the integrated operating or hybrid room of the healthcare institution, said core unit being configured to output at least a video signal and at least a data signal; synchronous multi-track registration means connected to said core unit and configured to record and transmit said data/and or said video signal; a plurality of display units, each connected to said core unit and configured to display said video signal and/or a visual representation of said data signal in one or more display windows.

2. Apparatus according to the previous claim, wherein said core unit is configured to acquire at least an input signal from said medical unit and/or said engineering unit, said core unit also comprising a data elaboration module configured to acquire said input signals, extract at least an input value from each of said data signals and to compute at least an output value, said data signal being representative of said output value.

3. Apparatus according to any one of the foregoing claims, wherein the core unit also comprises a control module configured to output a control signal for at least one of said medical unit and/or display unit and/or engineering unit.

4. Apparatus according to any one of the foregoing claims, wherein the core unit is connectable to a network of the healthcare institution, preferably to an healthcare information system (HIS) containing the electronic patient records and/or to a laboratory information system (LIS) containing the results of the laboratory tests and or to a radiology information system (RIS) containing the static and dynamic images obtained during the patients' study.

5. Apparatus according to any one of the foregoing claims, wherein the core unit is connectable to the PACS system and/or to a central information repository of a healthcare institution. <please explain what PACS is>

6. Apparatus according to any one of the foregoing claims, wherein said synchronous multi-track registration means is configured to send a previously recorded data and/or video signal to the core unit, said core unit being configured to perform the function of a simultaneous multi-track player for reproducing synchronously and simultaneously said data/and sais video signal in order to display a previously recorded operation. <move to description>: containing information from the medical equipment, medical devices, engineering systems, as well as the types and time of information arrival from the HIS, LIS, RIS, and the PACS;

7. Apparatus according to any one of the foregoing claims, wherein said core unit comprises a report module configured to fill at least a predetermined reporting form.

8. Apparatus according to any one of the foregoing claims, wherein said core unit is configured for back-up control of said medical unit and/or display units and/or said engineering unit.

9. Apparatus according to any one of the foregoing claims, wherein said core unit is configured for performing the function of multi-track non-linear editing to create educational, and/or research materials, and/or medical routings, and/or standards of medical procedures performed in the surgical or hybrid operations.

10. Apparatus according to any one of the foregoing claims, wherein said core unit is placed beyond the clean zone of the operating room.

11. Apparatus according to any one of the foregoing claims, wherein said medical unit is at least one of:
- surgical site video camera;
- endoscope video camera;
- operating microscope video cameras;
- operating room general view video camera;
- ultrasound device;
- X-ray - angiographic device apparatus;
- devices for functional diagnostics;
- patient monitor;
- anesthetic equipment;
- fluid therapy station;
- heart-lung machine;
- lungmotor;
- medical analyzers.

12. Apparatus according to any one of the foregoing claims, wherein said engineering unit is at least one of:
- laminar flow and microclimate provision system;
- temperature control system;
- gas supply system;
- power supply system.

13. Apparatus according to any one of the foregoing claims, wherein said core unit is configured to evaluate the composition and flow rate of air and medical gases, temperature, humidity, voltage applied to the medical devices and equipment at the same time as the parameters are measured.

14. Apparatus according to any one of the foregoing claims, wherein it comprises in the operating room one of said display units for each member of the surgical team. <move to description>: the individual displays are located on the outboard brackets adapted to change the location, as well as in a wall or the shelves of the health pendants.

15. Apparatus according to any one of the foregoing claims, wherein it comprises a cable system connecting said core unit to a medical unit and/or engineering unit and/or display unit; said cable system being located inside pendants, brackets, suspended ceilings and walls of the room, the cable outlets being located on the heath pendants on the walls or ceiling of the operating room.

16. Apparatus according to any one of the foregoing claims, wherein said core unit is configured to connect to the hardware and software system of physicians, consultants, experts, health care managers, training rooms and conference rooms; said systems being configured to remotely access the operating room system for consultations and remote medical training, including the on-line as well as view of the previous operations records.
